# EUROPÄISCHE PATENTSCHRIFT

(11) **EP 1 095 028 B1**
(45) Veröffentlichungstag und Bekanntmachung des Hinweises auf die Patenterteilung: **07.09.2005**
(21) Anmeldenummer: 99934603.4
(22) Anmeldetag: 07.07.1999
(51) Int. Cl.: C07D 239/54, A01N 43/54

(54) **SUBSTITUIERTE ACYLAMINOPHENYL-URACILE**
SUBSTITUTED ACYLAMINO PHENYL URACILS
URACILES D'ACYLAMINOPHENYLE SUBSTITUEES

(30) Priorität: 09.07.1998 DE 19830694
(43) Veröffentlichungstag der Anmeldung: 02.05.2001
(73) Patentinhaber: Bayer CropScience AG, 40789 Monheim (DE)
(72) Erfinder: ANDREE, Roland, D-40764 Langenfeld (DE); DREWES, Mark, Wilhelm, D-40764 Langenfeld (DE); FEUCHT, Dieter, D-40789 Monheim (DE); PONTZEN, Rolf, D-42799 Leichlingen (DE); WETCHOLOWSKY, Ingo, CEP-13280-000 Vinhedo, SP (BR)
(86) Internationale Anmeldenummer: PCT/EP1999/004743
(87) Internationale Veröffentlichungsnummer: WO 2000/002867

(56) Entgegenhaltungen:
- EP-A- 0 563 384
- DE-A- 19 523 640

## Beschreibung

Die Erfindung betrifft neue substituierte Acylaminophenyl-uracile, Verfahren zu ihrer Herstellung und ihre Verwendung als Herbizide.

Bestimmte substituierte Aryluracile sind bereits aus der (Patent-)Literatur bekannt (vgl. EP-A-408382 / US-A-5084084 / US-A-5127935 / US-A-5154755, EP-A-563384 / US-A-5356863, WO-A-95/29168, WO-A-96/35679, WO-A-97/01542, WO-A-97/09319, WO-A-98/06706). Auch in DE 195 23 640 Al werden bereits bestimmte substituierte Carbonylaminophenyluracile beschrieben. Diese Verbindungen haben jedoch bisher keine besondere Bedeutung erlangt.

Es wurden nun neue substituierte Acylaminophenyl-uracile der allgemeinen Formel (I) 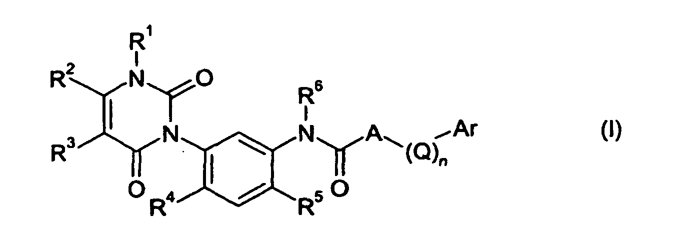 in welcher
- n: für die Zahlen 0 oder 1 steht,
- A: für jeweils gegebenenfalls durch Halogen substituiertes Alkandiyl (Alkylen) mit 1 bis 6 Kohlenstoffatomen oder Cycloalkandiyl mit 3 bis 6 Kohlenstoffatomen oder - für den Fall, daß dann n für 1 steht - auch für eine Einfachbindung oder ist mit Ar über eine Alkandiyl-Gruppierung mit 1 bis 3 Kohlenstoffatomen verknüpft ist,
- Ar: für jeweils substituiertes Phenyl, Naphthyl oder Benzyl steht, wobei die Substituenten der folgenden Aufzählung zu entnehmen sind:
Nitro, Cyano, Fluor, Chlor, Brom, Methyl, Ethyl, n- oder i-Propyl, n-, i-, s- oder t-Butyl Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n- oder i-Propoxycarbonyl steht,

- Q: für O (Sauerstoff), S (Schwefel), SO, SO₂, NH oder N(C₁-C₄-Alkyl) steht,
- R¹: für Wasserstoff, Amino oder gegebenenfalls durch Cyano, Carboxy, Halogen, C₁-C₄-Alkoxy oder C₁-C₄-Alkoxy-carbonyl substituiertes Alkyl mit 1 bis 6 Kohlenstoffatomen steht,
- R²: für Carboxy, Cyano, Carbamoyl, Thiocarbamoyl oder für jeweils gegebenenfalls durch Cyano, Halogen oder C₁-C₄-Alkoxy substituiertes Alkyl oder Alkoxycarbonyl mit jeweils 1 bis 6 Kohlenstoffatomen in den Alkylgruppen steht,
- R³: für Wasserstoff, Halogen oder gegebenenfalls durch Halogen substituiertes Alkyl mit 1 bis 6 Kohlenstoffatomen steht,
- R⁴: für Wasserstoff, Cyano, Carbamoyl, Thiocarbamoyl oder Halogen steht,
- R⁵: für Cyano, Carbamoyl, Thiocarbamoyl, Halogen, oder für jeweils gegebenenfalls durch Halogen substituiertes Alkyl oder Alkoxy mit jeweils 1 bis 4 Kohlenstoffatomen steht, und
- R⁶: für jeweils gegebenenfalls durch Halogen substituiertes Alkylsulfonyl mit 1 bis 6 Kohlenstoffatomen steht,
gefunden.

In den Definitionen sind die Kohlenwasserstoffketten, wie Alkyl oder Alkandiyl - auch in Verbindung mit Heteroatomen, wie in Alkoxy - jeweils geradkettig oder verzweigt.

Soweit die erfindungsgemäßen Verbindungen der allgemeinen Formel (I) asymmetrisch substituierte Kohlenstoffatome enthalten, betrifft die Erfindung jeweils die R-Enantiomeren und die S-Enantiomeren sowie beliebige Mischungen dieser Enantiomeren, insbesondere die Racemate.
- A: steht bevorzugt für jeweils gegebenenfalls durch Fluor und/oder Chlor substituiertes Methylen, Ethan-1,1-diyl (Ethyliden), Ethan-1,2-diyl (Dimethylen), Propan-1,1-diyl (Propyliden), Propan-1,2-diyl oder Propan-1,3-diyl (Trimethylen), Cyclopropan-1,1-diyl, Cyclopropan-1,2-diyl, Cyclobutan-1,1-diyl, Cyclopentan-1,1-diyl oder Cyclohexan-1,1-diyl, oder - für den Fall, daß dann n für 1 steht - auch für eine Einfachbindung, oder ist mit Ar über eine 1,2-Ethandiyl-(Dimethylen)-Gruppierung verknüpft.
- Ar: steht bevorzugt für jeweils substituiertes Phenyl oder Naphthyl, wobei die Substituenten der folgenden Aufzählung zu entnehmen sind:
Nitro, Cyano, Fluor, Chlor, Brom, Methyl, Ethyl, n- oder i-Propyl, n-, i-, s- oder t-Butyl, Trifluonnethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n- oder i-Propoxycarbonyl.
- Q: steht bevorzugt für O (Sauerstoff), S (Schwefel), SO, SO₂, NH oder N(Methyl).
- R¹: steht bevorzugt für Wasserstoff, Amino oder jeweils gegebenenfalls durch Cyano, Fluor, Chlor, Methoxy oder Ethoxy substituiertes Methyl oder Ethyl.
- R²: steht bevorzugt für Carboxy, Cyano, Carbamoyl, Thiocarbamoyl oder für jeweils gegebenenfalls durch Cyano, Fluor, Chlor, Methoxy oder Ethoxy substituiertes Methyl, Ethyl, n- oder i-Propyl, Methoxycarbonyl oder Ethoxycarbonyl.
- R³: steht bevorzugt für Wasserstoff, Fluor, Chlor, Brom oder jeweils gegebenenfalls durch Fluor und/oder Chlor substituiertes Methyl, Ethyl, n- oder i-Propyl.
- R⁴: steht bevorzugt für Wasserstoff, Fluor oder Chlor.
- R⁵: steht bevorzugt für Cyano, Carbamoyl, Thiocarbamoyl, Fluor, Chlor, Brom, oder für jeweils gegebenenfalls durch Fluor und/oder Chlor substituiertes Methyl, Ethyl, n- oder i-Propyl, Methoxy, Ethoxy, n- oder i-Propoxy.
- R⁶: steht bevorzugt für jeweils gegebenenfalls durch Fluor und/oder Chlor substituiertes Methylsulfonyl, Ethylsulfonyl, n- oder i-Propylsulfonyl oder n-, i-, s- oder t-Butylsulfonyl.
- A: steht besonders bevorzugt für Methylen, Ethan-1,1-diyl (Ethyliden) oder Ethan-1,2-diyl (Dimethylen).
- Q: steht besonders bevorzugt für O (Sauerstoff).
- R¹: steht besonders bevorzugt für Wasserstoff, Amino oder Methyl.
- R²: steht besonders bevorzugt für Trifluormethyl.
- R³: steht besonders bevorzugt für Wasserstoff, Chlor oder Methyl.
- R⁵: steht besonders bevorzugt für Cyano, Carbamoyl, Thiocarbamoyl, Chlor oder Brom.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Erfindungsgemäß bevorzugt sind die Verbindungen der Formel (I), in welchen eine Kombination der vorstehend als bevorzugt aufgeführten Bedeutungen vorliegt.

Erfindungsgemäß besonders bevorzugt sind die Verbindungen der Formel (I), in welchen eine Kombination der vorstehend als besonders bevorzugt aufgeführten Bedeutungen vorliegt.

Beispiele für die erfindungsgemäßen Verbindungen der allgemeinen Formel (I) sind in den nachstehenden Gruppen aufgeführt.

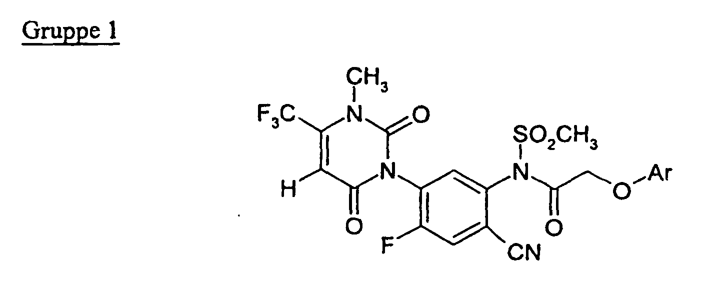

Ar hat dabei die in der nachstehenden Auflistung angegebenen Bedeutungen:
2-Cyano-phenyl, 3-Cyano-phenyl, 4-Cyano-phenyl, 2-Fluor-phenyl, 3-Fluor-phenyl, 4-Fluor-phenyl, 2,3-Difluor-phenyl, 2,4-Difluor-phenyl, 2,5-Difluor-phenyl, 2,6-Difluor-phenyl, 3,4-Difluor-phenyl, 3,5-Difluor-phenyl, 2-Chlor-phenyl, 3-Chlor-phenyl, 4-Chlor-phenyl, 2,3-Dichlor-phenyl, 2,4-Dichlor-phenyl, 2,5-Dichlor-phenyl, 2,6-Dichlor-phenyl, 3,4-Dichlor-phenyl, 3,5-Dichlor-phenyl, 2-Brom-phenyl, 3-Brom-phenyl, 4-Brom-phenyl, 2-Methyl-phenyl, 3-Methyl-phenyl, 4-Methyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 2-Trifluormethyl-phenyl, 3-Trifluormethyl-phenyl, 4-Trifluormethyl-phenyl, 2-Methoxy-phenyl, 3-Methoxy-phenyl, 4-Methoxy-phenyl, 2,4-Dimethoxy-phenyl, 2,5-Dimethoxy-phenyl, 2,6-Dimethoxy-phenyl, 3,4-Dimethoxy-phenyl, 2-Difluormethoxy-phenyl, 4-Difluormethoxy-phenyl, 2-Trifluonnethoxy-phenyl, 4-Trifluormethoxy-phenyl, 2-Chlor-4-methyl-phenyl, 4-Chlor-2-methyl-phenyl, 2-Fluor-4-chlor-phenyl, 2-Chlor-4-fluor-phenyl, 2-Chlor-4-brom-phenyl, 2-Brom-4-chlor-phenyl, 2-Fluor-4-brom-phenyl, 2-Brom-4-fluor-phenyl, 4-Fluor-2-methyl-phenyl, 4-Brom-2-methyl-phenyl.

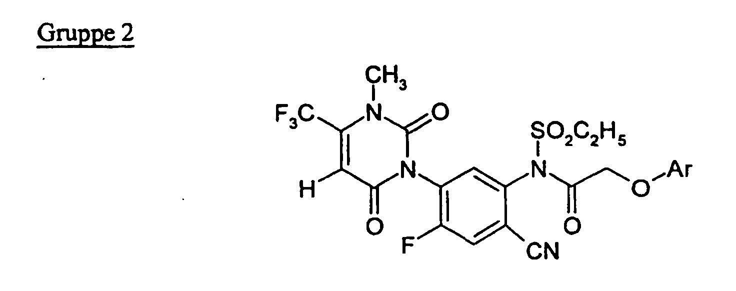

Ar hat dabei die oben in Gruppe 1 angegebenen Bedeutungen

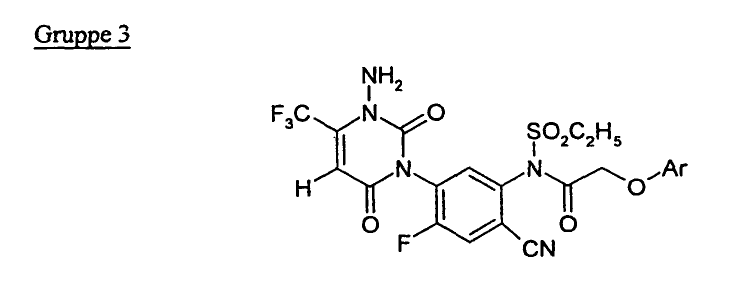

Ar hat dabei die oben in Gruppe 1 angegebenen Bedeutungen

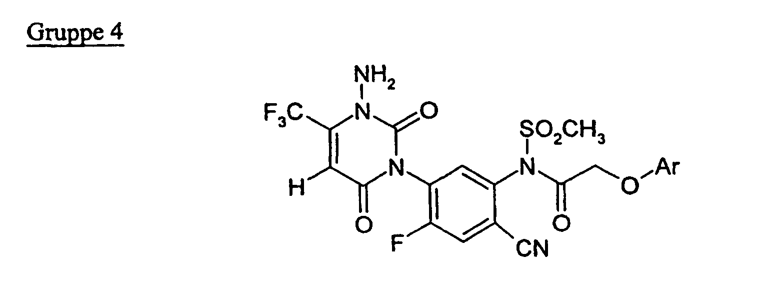

Ar hat dabei die oben in Gruppe 1 angegebenen Bedeutungen

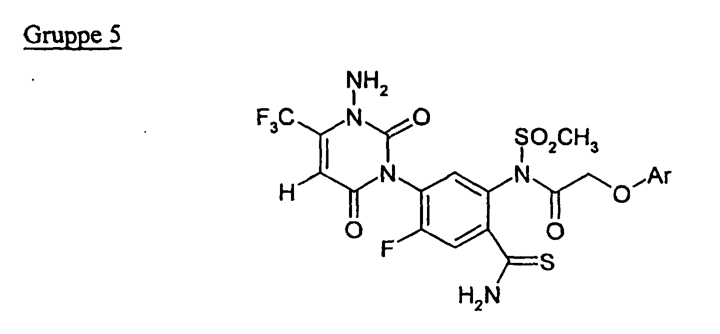

Ar hat dabei die oben in Gruppe 1 angegebenen Bedeutungen

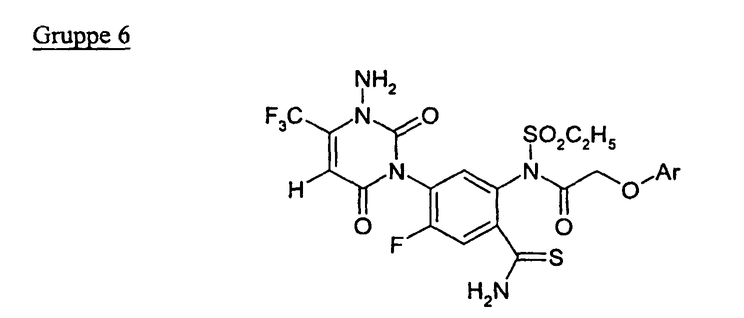

Ar hat dabei die oben in Gruppe 1 angegebenen Bedeutungen

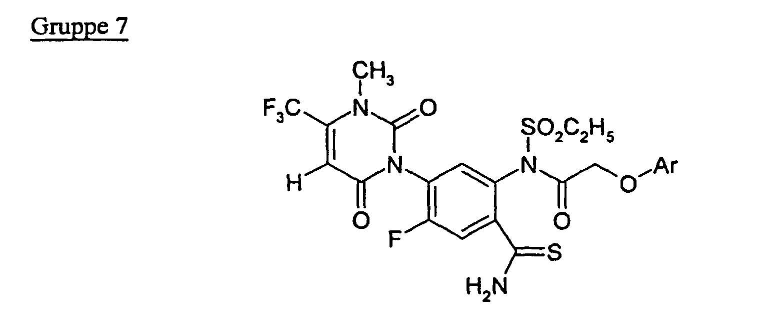

Ar hat dabei die oben in Gruppe 1 angegebenen Bedeutungen

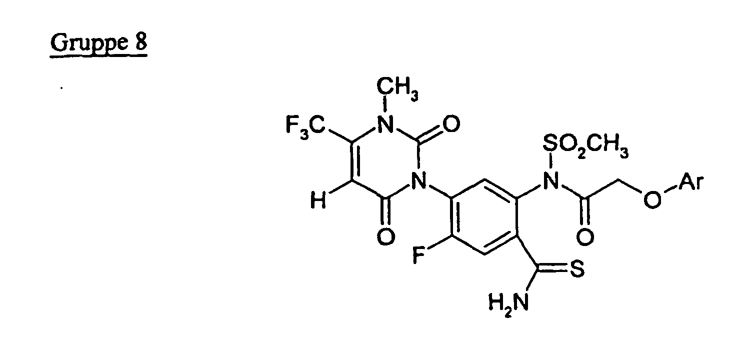

Ar hat dabei die oben in Gruppe 1 angegebenen Bedeutungen

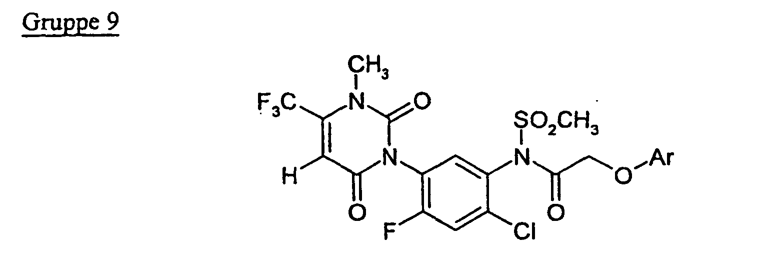

Ar hat dabei die oben in Gruppe 1 angegebenen Bedeutungen

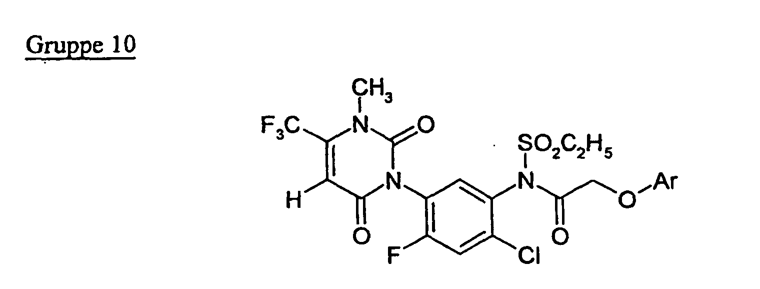

Ar hat dabei die oben in Gruppe 1 angegebenen Bedeutungen

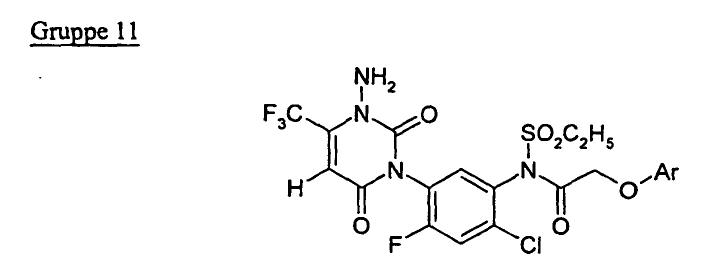

Ar hat dabei die oben in Gruppe 1 angegebenen Bedeutungen

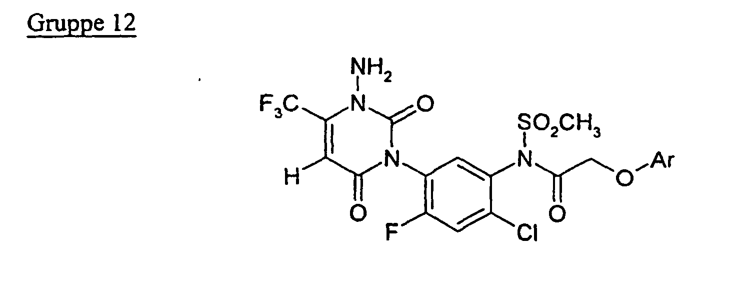

Ar hat dabei die oben in Gruppe 1 angegebenen Bedeutungen

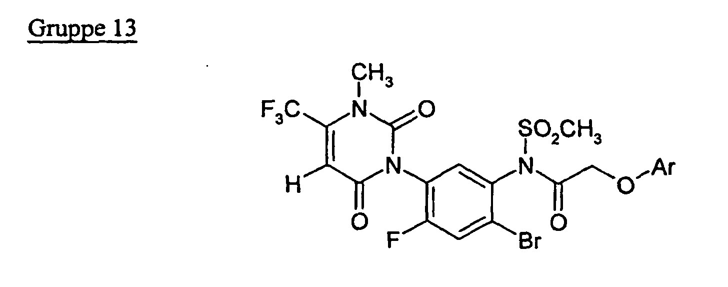

Ar hat dabei die oben in Gruppe 1 angegebenen Bedeutungen

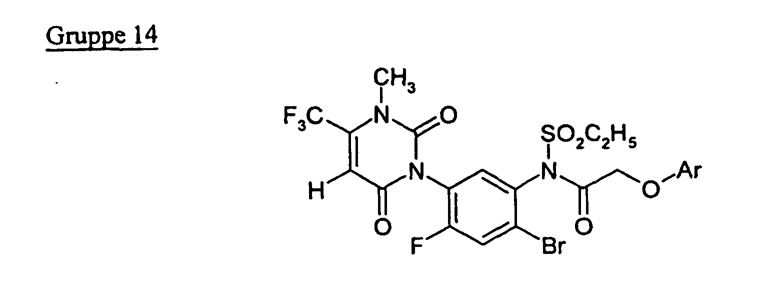

Ar hat dabei die oben in Gruppe 1 angegebenen Bedeutungen

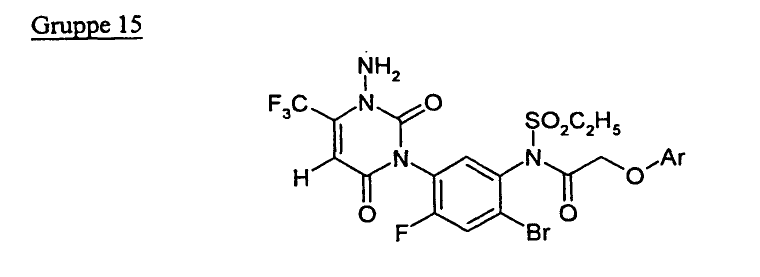

Ar hat dabei die oben in Gruppe 1 angegebenen Bedeutungen

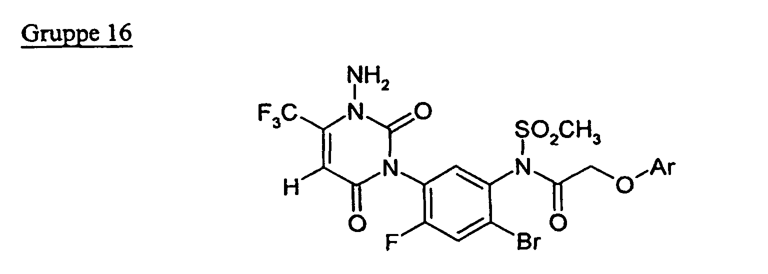

Ar hat dabei die oben in Gruppe 1 angegebenen Bedeutungen

Die neuen substituierten Acylaminophenyl-uracile der allgemeinen Formel (I) weisen interessante biologische Eigenschaften auf. Sie zeichnen sich insbesondere durch starke herbizide Wirksamkeit aus.

Man erhält die neuen substituierten Acylaminophenyl-uracile der allgemeinen Formel (I), wenn man Aminophenyl-uracile der allgemeinen Formel (II) 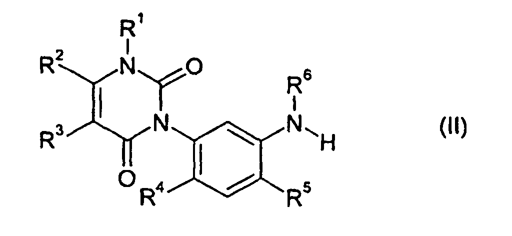 in welcher
R¹, R², R³, R⁴, R⁵ und R⁶ die oben angegebene Bedeutung haben,
mit Acylierungsmitteln der allgemeinen Formel (III) 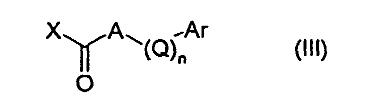 in welcher
- n, A, Ar und Q: die oben angegebene Bedeutung haben, und
- x: für Halogen steht,
gegebenenfalls in Gegenwart eines Reaktionshilfsmittels und gegebenenfalls in Gegenwart eines Verdünnungsmittels umsetzt,
und gegebenenfalls im Anschluß daran im Rahmen der Substituentendefinition auf übliche Weise elektrophile oder nucleophile bzw. Oxidations- oder Reduktionsreaktionen durchführt.

Die Verbindungen der allgemeinen Formel (I) können nach üblichen Methoden in andere Verbindungen der allgemeinen Formel (I) gemäß obiger Definition umgewandelt werden, beispielsweise durch Aminierung oder Alkylierung (z.B. R¹: H → NH₂, CH₃, Umsetzung mit Dicyan bzw. Hydrogensulfid (z.B. R⁵: Br → CN, CN → CSNH₂, vgl. die Herstellungsbeispiele).

Verwendet man beispielsweise 1-(2,4-Dichlor-5-methylsulfonylamino-phenyl)-4-difluonnethyl-3,6-dihydro-2,6-dioxo-1(2H)-pyrimidin und (4-Chlor-2-methyl-phenoxy)-acetylchlorid als Ausgangsstoffe, so kann der Reaktionsablauf beim erfindungsgemäßen Verfahren durch das folgende Formelschema skizziert werden:

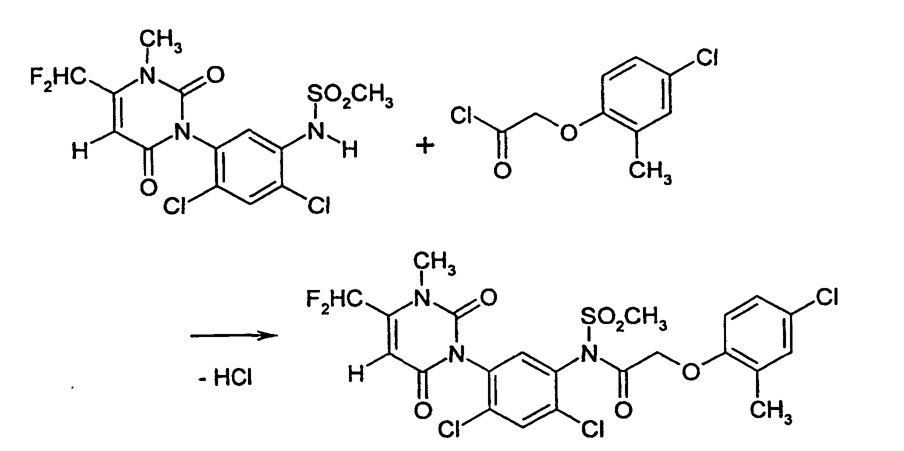

Die beim erfindungsgemäßen Verfahren zur Herstellung von Verbindungen der Formel (I) als Ausgangsstoffe zu verwendenden Aminophenyluracile sind durch die Formel (II) allgemein definiert. In der Formel (II) haben R¹, R², R³, R⁴, R⁵ und R⁶ vorzugsweise diejenigen Bedeutungen, die bereits oben im Zusammenhang mit der Beschreibung der erfindungsgemäßen Verbindungen der Formel (I) als bevorzugt, besonders bevorzugt oder ganz besonders bevorzugt für R¹, R², R³, R⁴, R⁵ und R⁶ angegeben wurden.

Die Ausgangsstoffe der allgemeinen Formel (II) sind bekannt und/oder können nach an sich bekannten Verfahren hergestellt werden (vgl. EP-A-408382, EP-A-648749, WO-A-97/01542).

Die beim erfindungsgemäßen Verfahren weiter als Ausgangsstoffe zu verwendenden Acylierungsmittel sind durch die Formel (III) allgemein definiert. In der Formel (III) haben n, A, Ar und Q vorzugsweise diejenigen Bedeutungen, die bereits oben im Zusammenhang mit der Beschreibung der erfindungsgemäßen Verbindungen der Formel (I) als bevorzugt, besonders bevorzugt, ganz besonders bevorzugt oder am meistens bevorzugt für n, A, Ar und Q angegeben wurden; X steht vorzugsweise für Fluor, Chlor oder Brom, insbesondere für Chlor.

Die Ausgangsstoffe der allgemeinen Formel (III) sind bekannte Synthesechemikalien.

Das erfindungsgemäße Verfahren zur Herstellung der Verbindungen der allgemeinen Formel (I) wird vorzugsweise unter Verwendung eines Reaktionshilfsmittels durchgeführt. Als Reaktionshilfsmittel für das erfindungsgemäße Verfahren kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder - alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium-oder Calcium-hydrid, Lithium-, Natrium-, Kalium- oder Calcium-hydroxid, Natrium-oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Das erfindungsgemäße Verfahren zur Herstellung der Verbindungen der allgemeinen Formel (I) wird vorzugsweise unter Verwendung eines Verdünnungsmittels durchgerührt. Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von monokotylen und dikotylen Unkräutern sowohl im Vorauflauf- als auch im Nachauflauf-Verfahren. Sie zeigen hohe herbizide Aktivität und ein breites Wirkungsspektrum bei Anwendung auf dem Boden und auf oberirdische Pflanzenteile.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Venvendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clethodim, Clodina-fop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

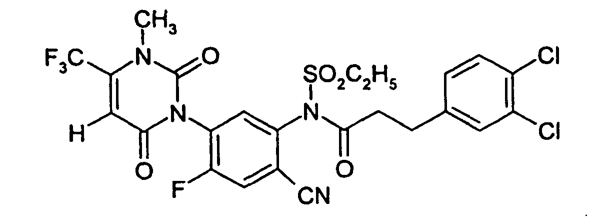

Eine Mischung aus 2,1g (5 mmol) 1-(4-Cyano-5-ethylsulfonylamino-2-fluor-phenyl)-3-methyl-4-trifluormethyl-3,6-dihydro-2,6-dioxo-1(2H)-pyrimidin, 1,42 g (5 mMol) 3-(3,4-Dichlor-phenyl)-propansäurechlorid, 0,63 g Triethylamin und 50 ml Acetonitril wird 3 Stunden bei Raumtemperatur (ca. 20°C) gerührt und anschließend im Wasserstrahlvakuum eingeengt. Der Rückstand wird in Chloroform aufgenommen und das kristallin anfallende Produkt durch Absaugen isoliert (Produktfraktion 1). Die Mutterlauge wird mit 1N-Salzsäure und dann mit Wasser gewaschen, mit Natriumsulfat getrocknet und filtriert. Das Filtrat wird im Wasserstrahlvakuum eingeengt, der Rückstand mit Essigsäureethylester und Diethylether digeriert und das kristallin anfallende Produkt durch Absaugen isoliert (Produktfraktion 2). Die beiden Produktfraktionen werden vereinigt und mit 1N-Salzsäure und Essigsäureethylester verrührt. Die wässrige Phase wird zweimal mit Essigsäureethylester extrahiert, die vereinigten organischen Phasen mit Natriumsulfat getrocknet und filtriert. Das Filtrat wird im Wasserstrahlvakuum eingeengt, der Rückstand mit Diethylether digeriert und das kristallin angefallene Produkt durch Absaugen isoliert.

Man erhält 1,3 g (42% der Theorie) 1-[4-Cyano-5-(N-ethylsulfonyl-N-(3-(3,4-dichlor-phenyl)-propanoyl-amino)-2-fluor-phenyl]-3-methyl-4-trifluonnethyl-3,6-dihydro-2,6-dioxo-1(2H)-pyrimidin vom Schmelzpunkt 205°C.

Analog zu Herstellungsbeispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

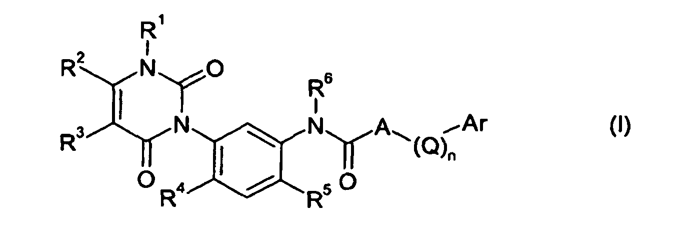

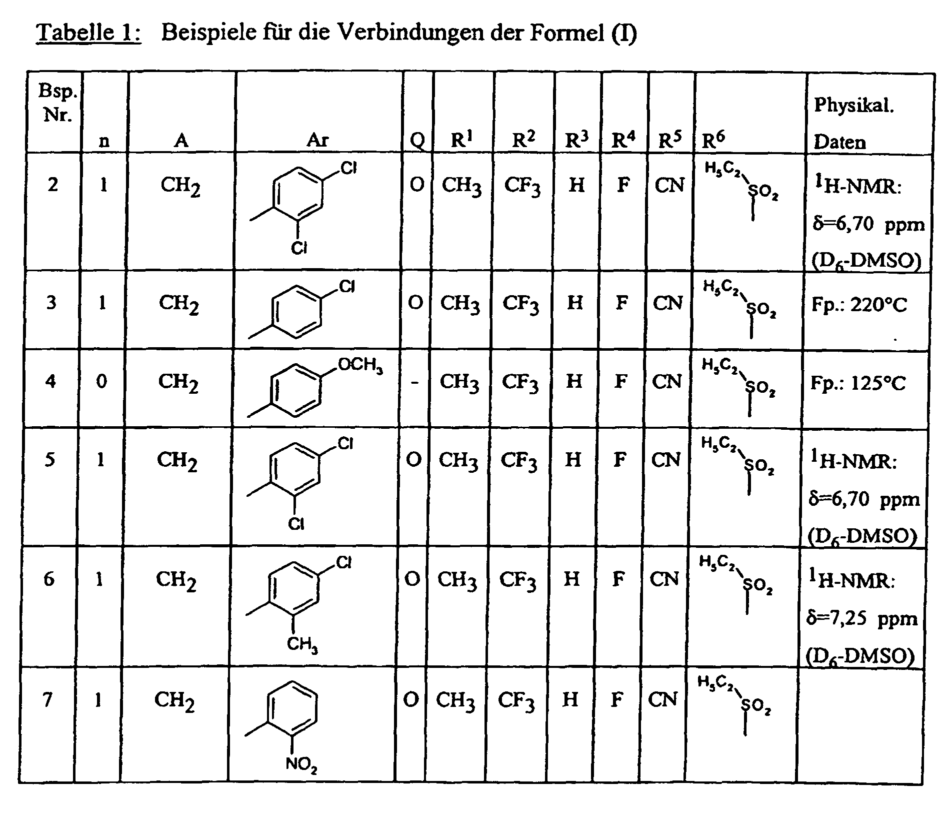

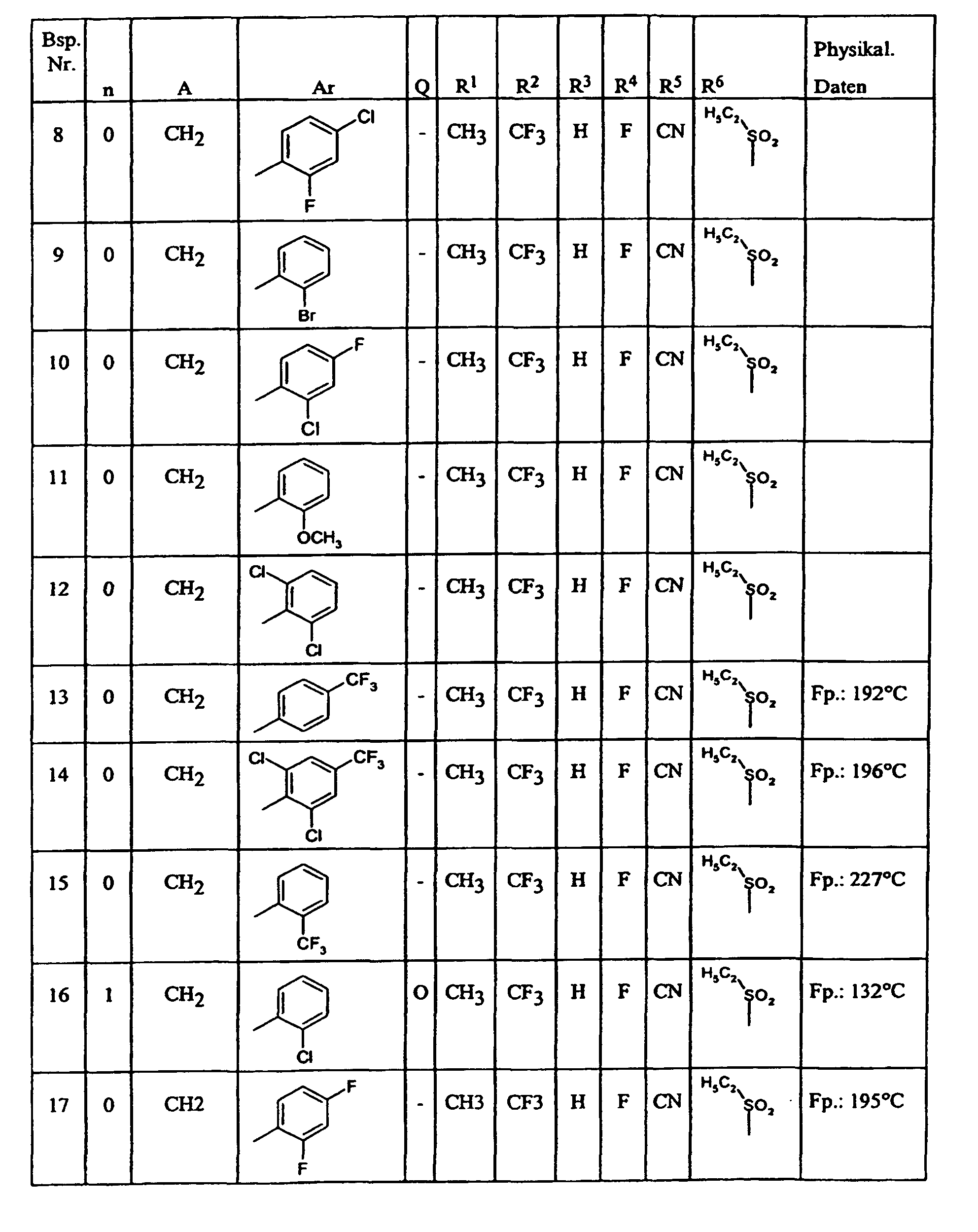

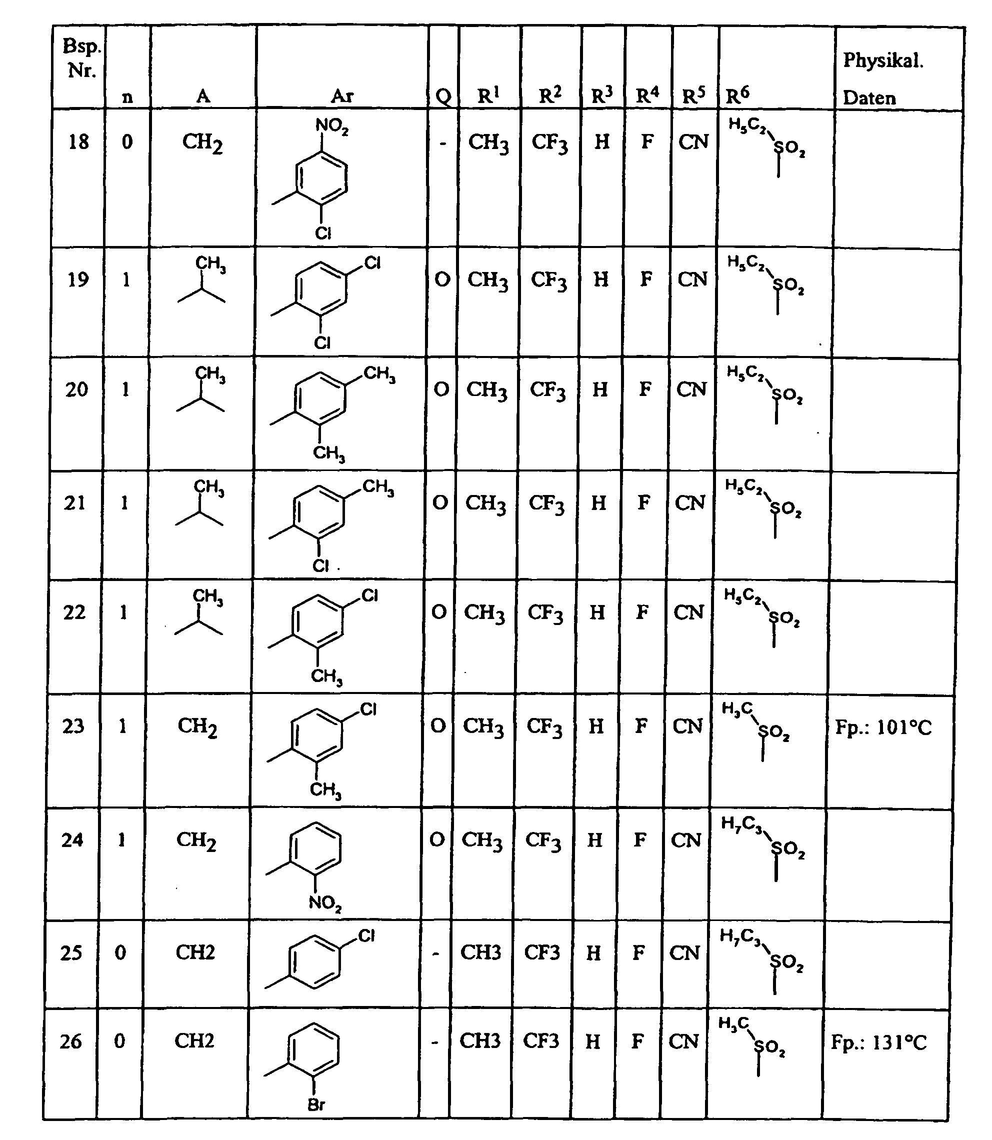

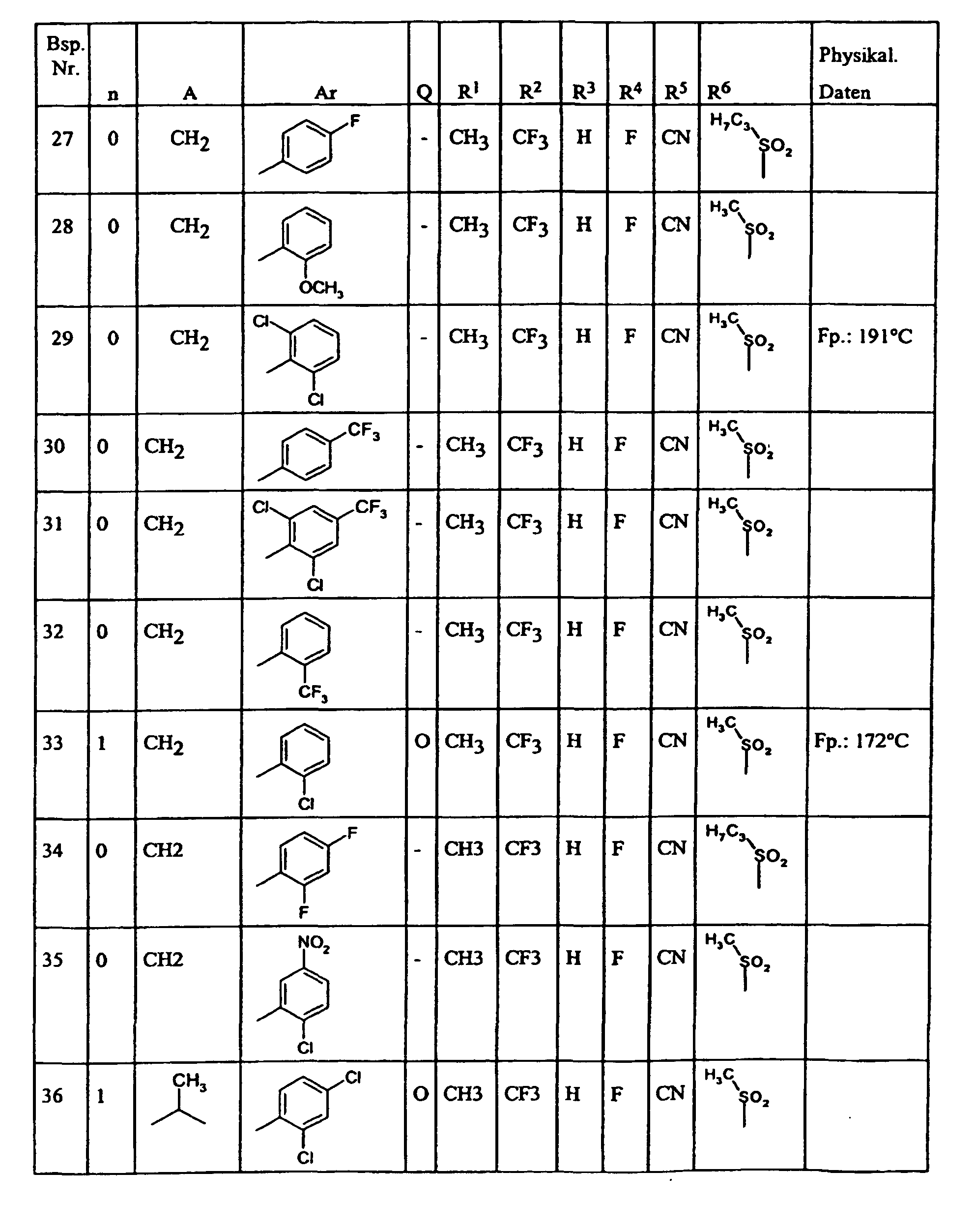

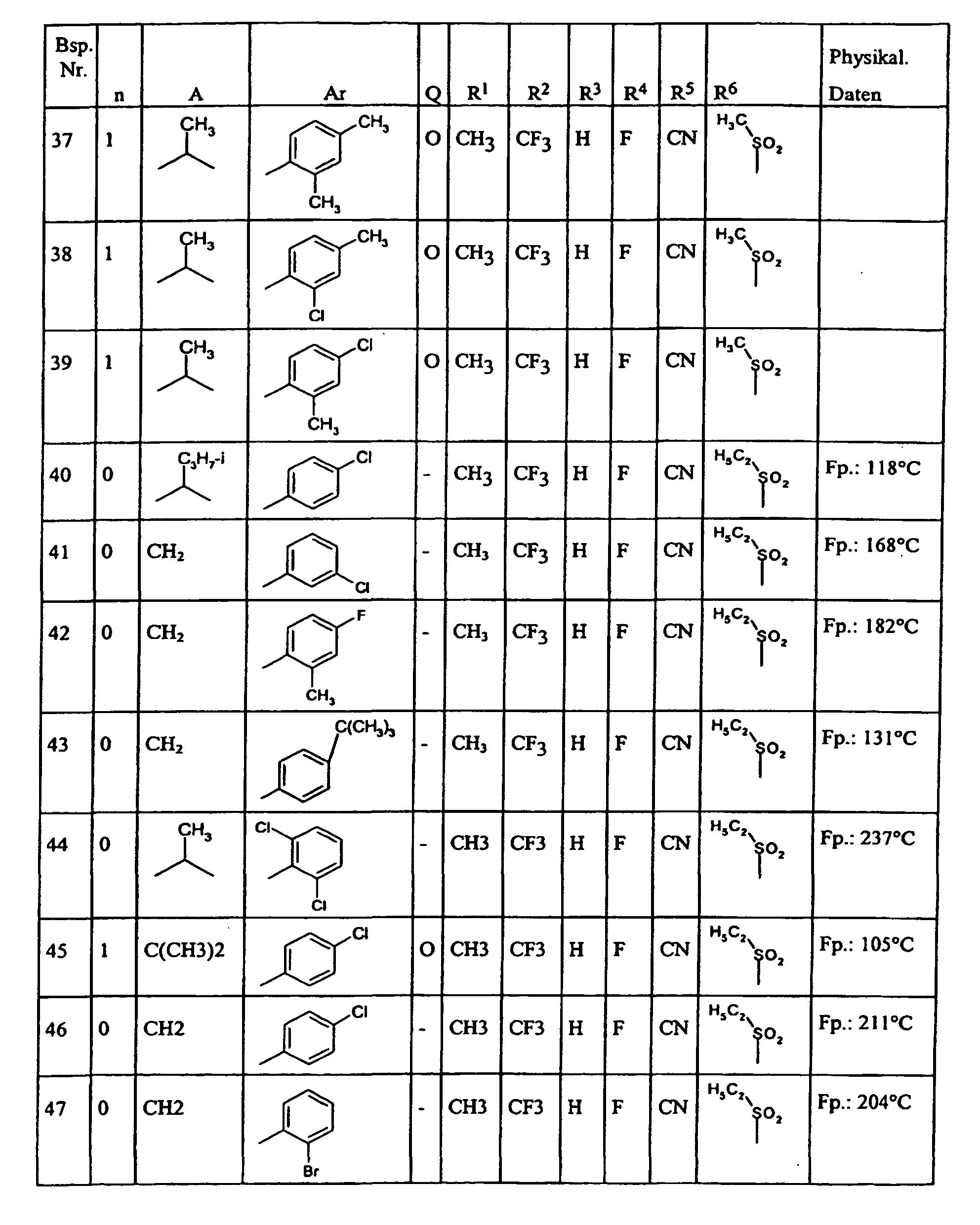

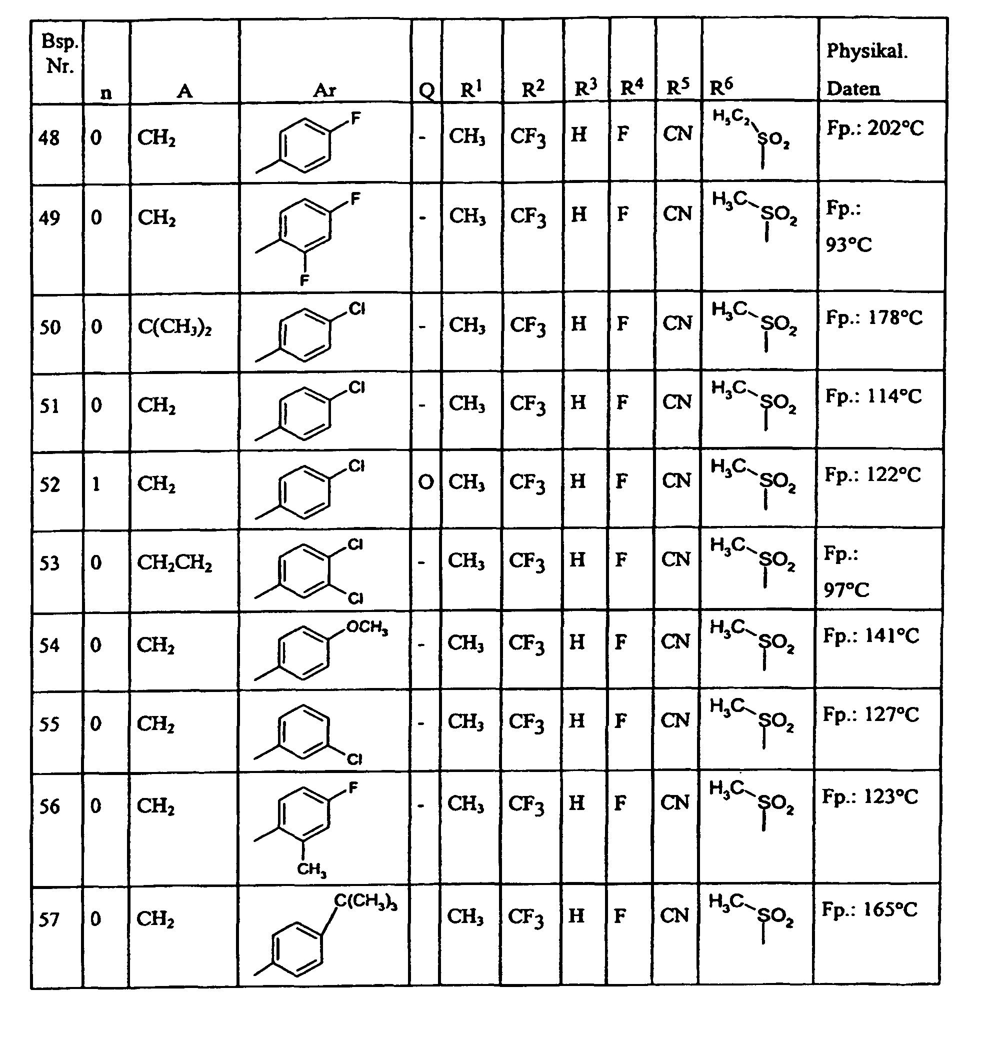

### Anwendungsbeipiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt beispielsweise die Verbindung gemäß Herstellungsbeispiel 2 sehr starke Wirkung gegen Unkräuter.

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt beispielsweise die Verbindung gemäß Herstellungsbeispiel 2 sehr starke Wirkung gegen Unkräuter.

## Patentansprüche

1. Substituierte Acylaminophenyl-uracile der allgemeinen Formel (I) 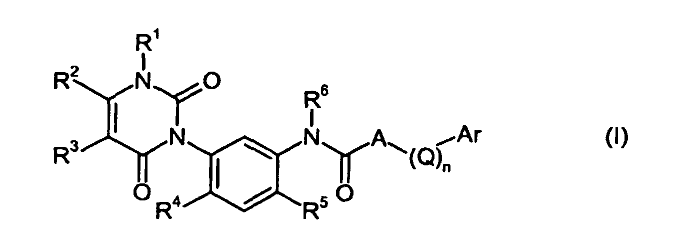 in welcher
n für die Zahlen 0 oder 1 steht,
A für jeweils gegebenenfalls durch Halogen substituiertes Alkandiyl (Alkylen) mit 1 bis 6 Kohlenstoffatomen oder Cycloalkandiyl mit 3 bis 6 Kohlenstoffatomen steht, oder - für den Fall, daß dann n für 1 steht - auch für eine Einfachbindung steht, oder mit Ar über eine Alkandiyl-Gruppierung mit 1 bis 3 Kohlenstoffatomen verknüpft ist,
Ar für jeweils substituiertes Phenyl, Naphthyl oder Benzyl steht, wobei die Substituenten der folgenden Aufzählung zu entnehmen sind:
Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n-oder i-Propylthio, Difluormethylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n- oder i-Propoxycarbonyl,
Q für O (Sauerstoff), S (Schwefel), SO, SO₂, NH oder N(C₁-C₄-Alkyl) steht,
R¹ für Wasserstoff, Amino oder gegebenenfalls durch Cyano, Carboxy, Halogen, C₁-C₄-Alkoxy oder C₁-C₄-Alkoxy-carbonyl substituiertes Alkyl mit 1 bis 6 Kohlenstoffatomen steht,
R² für Carboxy, Cyano, Carbamoyl, Thiocarbamoyl oder für jeweils gegebenenfalls durch Cyano, Halogen oder C₁-C₄-Alkoxy substituiertes Alkyl oder Alkoxycarbonyl mit jeweils 1 bis 6 Kohlenstoffatomen in den Alkylgruppen steht,
R³ für Wasserstoff, Halogen oder gegebenenfalls durch Halogen substituiertes Alkyl mit 1 bis 6 Kohlenstoffatomen steht,
R⁴ für Wasserstoff, Cyano, Carbamoyl, Thiocarbamoyl oder Halogen steht,
R⁵ für Cyano, Carbamoyl, Thiocarbamoyl, Halogen, oder für jeweils gegebenenfalls durch Halogen substituiertes Alkyl oder Alkoxy mit jeweils 1 bis 4 Kohlenstoffatomen steht, und
R⁶ für jeweils gegebenenfalls durch Halogen substituiertes Alkylsulfonyl mit 1 bis 6 Kohlenstoffatomen steht.

2. Substituierte Acylaminophenyl-uracile gemäß Anspruch 1, **dadurch gekennzeichnet, dass**
n für die Zahlen 0 oder 1 steht,
A für jeweils gegebenenfalls durch Fluor und/oder Chlor substituiertes Methylen, Ethan-1,1-diyl (Ethyliden), Ethan-1,2-diyl (Dimethylen), Propan-1,1-diyl (Propyliden), Propan-1,2-diyl oder Propan-1,3-diyl (Trimethylen), Cyclopropan-1,1-diyl, Cyclopropan-1,2-diyl, Cyclobutan-1,1-diyl, Cyclopentan-1,1-diyl oder Cyclohexan-1,1-diyl steht, oder - für den Fall, daß dann n für 1 steht - auch für eine Einfachbindung steht, oder mit Ar über eine 1,2-Ethandiyl-(Dimethylen)-Gruppierung verknüpft ist,
Ar für jeweils substituiertes Phenyl, Naphthyl oder Benzyl steht, wobei die Substituenten der folgenden Aufzählung zu entnehmen sind: Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n-oder i-Propylthio, Difluormethylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n- oder i-Propoxycarbonyl,
Q für O (Sauerstoff), S (Schwefel), SO, SO₂, NH oder N(Methyl) steht,
R¹ für Wasserstoff, Amino oder jeweils gegebenenfalls durch Cyano, Fluor, Chlor, Methoxy oder Ethoxy substituiertes Methyl oder Ethyl steht,
R² für Carboxy, Cyano, Carbamoyl, Thiocarbamoyl oder für jeweils gegebenenfalls durch Cyano, Fluor, Chlor, Methoxy oder Ethoxy substituiertes Methyl, Ethyl, n- oder i-Propyl, Methoxycarbonyl oder Ethoxycarbonyl steht,
R³ für Wasserstoff, Fluor, Chlor, Brom oder jeweils gegebenenfalls durch Fluor und/oder Chlor substituiertes Methyl, Ethyl, n- oder i-Propyl steht,
R⁴ für Wasserstoff, Cyano, Carbamoyl, Thiocarbamoyl, Fluor oder Chlor steht,
R⁵ für Cyano, Carbamoyl, Thiocarbamoyl, Fluor, Chlor, Brom, oder für jeweils gegebenenfalls durch Fluor und/oder Chlor substituiertes Methyl, Ethyl, n- oder i-Propyl, Methoxy, Ethoxy, n- oder i-Propoxy steht, und
R⁶ für jeweils gegebenenfalls durch Fluor und/oder Chlor substituiertes Methylsulfonyl, Ethylsulfonyl, n- oder i-Propylsulfonyl oder n-, i-, s- oder t-Butylsulfonyl steht.

3. Substituierte Acylaminophenyl-uracile gemäß Anspruch 1, **dadurch gekennzeichnet, dass**
n für die Zahl 1 steht,
A für Methylen, Ethan-1,1-diyl (Ethyliden) oder Ethan-1,2-diyl (Dimethylen) steht,
Ar für jeweils substituiertes Phenyl, Naphthyl oder Benzyl steht,
wobei die Substituenten der folgenden Aufzählung zu entnehmen sind:
Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n-oder i-Propylthio, Difluormethylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n- oder i-Propoxycarbonyl,
Q für O (Sauerstoff)steht,
R¹ für Wasserstoff, Amino oder Methyl steht,
R² für Trifluormethyl steht,
R³ für Wasserstoff, Chlor oder Methyl steht,
R⁴ für Wasserstoff, Fluor oder Chlor steht,
R⁵ für Cyano, Carbamoyl, Thiocarbamoyl, Chlor oder Brom steht, und
R⁶ für jeweils gegebenenfalls durch Fluor und/oder Chlor substituiertes Methylsulfonyl, Ethylsulfonyl, n- oder i-Propylsulfonyl oder n-, i-, s-oder t-Butylsulfonyl steht.

4. Substituierte Acylaminophenyl-uracile gemäß Anspruch 1, **dadurch gekennzeichnet, dass**
n für die Zahl 0 steht,
A für Methylen, Ethan-1,1-diyl (Ethyliden) oder Ethan-1,2-diyl (Dimethylen) steht,
Ar für jeweils substituiertes Phenyl oder Naphthyl steht, wobei die Substituenten der folgenden Aufzählung zu entnehmen sind:
Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n-oder i-Propylthio, Difluormethylthio, Trifluormethylthio, Methylsulfinyl, Ethylsulfinyl, n- oder i-Propylsulfinyl, Trifluormethylsulfinyl, Methylsulfonyl, Ethylsulfonyl, n- oder i-Propylsulfonyl, Trifluormethylsulfonyl, Methoxycarbonyl, Ethoxycarbonyl, n- oder i-Propoxycarbonyl,
R¹ für Wasserstoff, Amino oder Methyl steht,
R² für Trifluormethyl steht,
R³ für Wasserstoff, Chlor oder Methyl steht,
R⁴ für Wasserstoff, Fluor oder Chlor steht,
R⁵ für Cyano, Carbamoyl, Thiocarbamoyl, Chlor oder Brom steht, und
R⁶ für jeweils gegebenenfalls durch Fluor und/oder Chlor substituiertes Methylsulfonyl, Ethylsulfonyl, n- oder i-Propylsulfonyl oder n-, i-, s-oder t-Butylsulfonyl steht.

5. Verfahren zum Herstellen von substituierten Acylaminophenyl-uracilen gemäß einem der Ansprüche 1 bis 4, **dadurch gekennzeichnet, dass** man Aminophenyl-uracile der allgemeinen Formel (II) 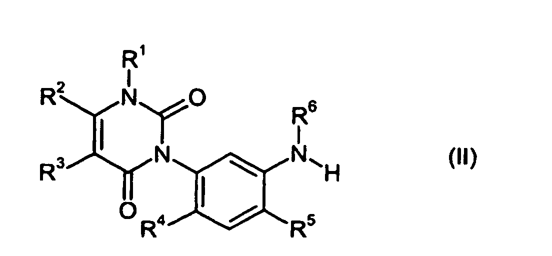 in welcher
R¹, R², R³, R⁴, R⁵ und R⁶ die in einem der Ansprüche 1 bis 4 angegebene Bedeutung haben,
mit Acylierungsmitteln der allgemeinen Formel (III) 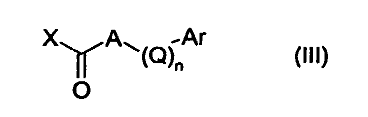 in welcher
n, A, Ar und Q die in einem der Ansprüche 1 bis 4 angegebene Bedeutung haben, und
X für Halogen steht,
gegebenenfalls in Gegenwart eines Reaktionshilfsmittels und gegebenenfalls in Gegenwart eines Verdünnungsmittels umsetzt,
und gegebenenfalls im Anschluß daran im Rahmen der Substituentendefinition auf übliche Weise elektrophile oder nucleophile bzw. Oxidations-oder Reduktionsreaktionen durchführt.

6. Verwendung von mindestens einem substituierten Acylaminophenyl-uracil gemäß einem der Ansprüche 1 bis 4 zur Bekämpfung von unerwünschten Pflanzen.

7. Herbizide Mittel, **gekennzeichnet durch** den Gehalt von mindestens einem substituierten Acylaminophenyl-uracil gemäß einem der Ansprüche 1 bis 4 und üblichen Streckmitteln.

## Claims

1. A substituted acylaminophenyl-uracil of the general formula (I) 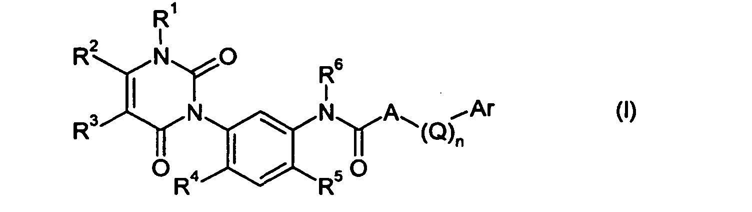 in which
n represents the numbers 0 or 1,
A represents in each case optionally halogen-substituted alkanediyl (alkylene) having 1 to 6 carbon atoms or cycloalkanediyl having 3 to 6 carbon atoms, or - in the event that n here represents 1 - also represents a single bond, or is linked to Ar via an alkanediyl group having 1 to 3 carbon atoms,
Ar represents in each case substituted phenyl, naphthyl or benzyl, the substituents being selected from the following list:
nitro, cyano, fluorine, chlorine, bromine, methyl, ethyl, n- or i-propyl, n-, i-, s- or t-butyl, trifluoromethyl, methoxy, ethoxy, n- or i-propoxy, difluoromethoxy, trifluoromethoxy, methylthio, ethylthio, n- or i-propylthio, difluoromethylthio, trifluoromethylthio, methylsulfinyl, ethylsulfinyl, n- or i-propylsulfinyl, trifluoromethylsulfinyl, methylsulfonyl, ethylsulfonyl, n- or i-propylsulfonyl, trifluoromethylsulfonyl, methoxycarbonyl, ethoxycarbonyl, n- or i-propoxycarbonyl,
Q represents O (oxygen), S (sulfur), SO, SO₂, NH or N(C₁-C₄-alkyl),
R¹ represents hydrogen, amino, or represents alkyl which has 1 to 6 carbon atoms and which is optionally substituted by cyano, carboxyl, halogen, C₁-C₄-alkoxy or C₁-C₄-alkoxycarbonyl,
R² represents carboxyl, cyano, carbamoyl, thiocarbamoyl, or represents alkyl or alkoxycarbonyl, each of which has 1 to 6 carbon atoms in the alkyl groups and each of which is optionally substituted by cyano, halogen or C₁-C₄-alkoxy,
R³ represents hydrogen, halogen or alkyl which has 1 to 6 carbon atoms and which is optionally substituted by halogen,
R⁴ represents hydrogen, cyano, carbamoyl, thiocarbamoyl or halogen,
R⁵ represents cyano, carbamoyl, thiocarbamoyl, halogen, or represents alkyl or alkoxy, each of which has 1 to 4 carbon atoms and each of which is optionally substituted by halogen, and
R⁶ represents alkylsulfonyl which has 1 to 6 carbon atoms and which is in each case optionally substituted by halogen.

2. A substituted acylaminophenyl-uracil as claimed in claim 1, wherein
n represents the numbers 0 or 1,
A represents methylene, ethane-1,1-diyl (ethylidene), ethane-1,2-diyl (dimethylene), propane-1,1-diyl (propylidene), propane-1,2-diyl or propane-1,3-diyl (trimethylene), cyclopropane-1,1-diyl, cyclopropane-1,2-diyl, cyclobutane-1,1-diyl, cyclopentane-1,1-diyl or cyclohexane-1,1-diyl, each of which is optionally substituted by fluorine and/or chlorine, or - in the event that n here represents 1 - also represents a single bond, or is linked to Ar via a 1,2-ethanediyl (dimethylene) group,
Ar represents in each case substituted phenyl, naphthyl or benzyl, the substituents being selected from the following list:
nitro, cyano, fluorine, chlorine, bromine, methyl, ethyl, n- or i-propyl, n-, i-, s- or t-butyl, trifluoromethyl, methoxy, ethoxy, n- or i-propoxy, difluoromethoxy, trifluoromethoxy, methylthio, ethylthio, n- or i-propylthio, difluoromethylthio, trifluoromethylthio, methylsulfinyl, ethylsulfinyl, n- or i- propylsulfinyl, trifluoromethylsulfinyl, methylsulfonyl, ethylsulfonyl, n- or i-propylsulfonyl, trifluoromethylsulfonyl, methoxycarbonyl, ethoxycarbonyl, n- or i-propoxycarbonyl,
Q represents O (oxygen), S (sulfur), SO, SO₂, NH or N(methyl),
R¹ represents hydrogen, amino, or represents methyl or ethyl, each of which is optionally substituted by cyano, fluorine, chlorine, methoxy or ethoxy,
R² represents carboxyl, cyano, carbamoyl, thiocarbamoyl, or represents methyl, ethyl, n- or i-propyl, methoxycarbonyl or ethoxycarbonyl, each of which is optionally substituted by cyano, fluorine, chlorine, methoxy or ethoxy,
R³ represents hydrogen, fluorine, chlorine, bromine, or represents methyl, ethyl, n- or i-propyl, each of which is optionally. substituted by fluorine and/or chlorine,
R⁴ represents hydrogen, cyano, carbamoyl, thiocarbamoyl, fluorine or chlorine,
R⁵ represents cyano, carbamoyl, thiocarbamoyl, fluorine, chlorine, bromine, or represents methyl, ethyl, n- or i-propyl, methoxy, ethoxy, n- or i-propoxy, each of which is optionally substituted by fluorine and/or chlorine, and
R⁶ represents methylsulfonyl, ethylsulfonyl, n- or i-propylsulfonyl or n-, i-, s- or t-butylsulfonyl, each of which is optionally substituted by fluorine and/or chlorine.

3. A substituted acylaminophenyl-uracil as claimed in claim 1, wherein
n represents the number 1,
A represents methylene, ethane-1,1-diyl (ethylidene) or ethane-1,2-diyl (dimethylene),
Ar represents in each case substituted phenyl, naphthyl or benzyl, where the substituents are selected from the following list:
nitro, cyano, fluorine, chlorine, bromine, methyl, ethyl, n- or i- propyl, n-, i-, s- or t-butyl, trifluoromethyl, methoxy, ethoxy, n- or i-propoxy, difluoromethoxy, trifluoromethoxy, methylthio, ethylthio, n- or i-propylthio, difluoromethylthio, trifluoromethylthio, methylsulfinyl, ethylsulfinyl, n- or i- propylsulfinyl, trifluoromethylsulfinyl, methylsulfonyl, ethylsulfonyl, n- or i-propylsulfonyl, trifluoromethylsulfonyl, methoxycarbonyl, ethoxycarbonyl, n- or i-propoxycarbonyl,
Q represents O (oxygen),
R¹ represents hydrogen, amino or methyl,
R² represents trifluoromethyl,
R³ represents hydrogen, chlorine or methyl,
R⁴ represents hydrogen, fluorine or chlorine,
R⁵ represents cyano, carbamoyl, thiocarbamoyl, chlorine or bromine,
R⁶ represents in each case optionally fluorine- and/or chlorine-substituted methylsulfonyl, ethylsulfonyl, n- or i-propylsulfonyl, n-, i- s- or t-butylsulfonyl.

4. A substituted acylaminophenyl-uracil as claimed in claim 1, wherein
n represents the number 0,
A represents methylene, ethane-1,1-diyl (ethylidene) or ethane-1,2-diyl (dimethylene),
Ar represents in each case substituted phenyl or naphthyl, where the substituents are selected from the following list:
nitro, cyano, fluorine, chlorine, bromine, methyl, ethyl, n- or i- propyl, n-, i-, s- or t-butyl, trifluoromethyl, methoxy, ethoxy, n- or i-propoxy, difluoromethoxy, trifluoromethoxy, methylthio, ethylthio, n- or i-propylthio, difluoromethylthio, trifluoromethylthio, methylsulfinyl, ethylsulfinyl, n- or i- propylsulfinyl, trifluoromethylsulfinyl, methylsulfonyl, ethylsulfonyl, n- or i-propylsulfonyl, trifluoromethylsulfonyl, methoxycarbonyl, ethoxycarbonyl, n- or i-propoxycarbonyl,
R¹ represents hydrogen, amino or methyl,
R² represents trifluoromethyl,
R³ represents hydrogen, chlorine or methyl,
R⁴ represents hydrogen, fluorine or chlorine,
R⁵ represents cyano, carbamoyl, thiocarbamoyl, chlorine or bromine, and
R⁶ represents in each case optionally fluorine- and/or chlorine-substituted methylsulfonyl, ethylsulfonyl, n- or i-propylsulfonyl, n-, i-, s- or t-butylsulfonyl.

5. A process for preparing a substituted acylaminophenyl-uracil as claimed in any of claims 1 to 4, wherein aminophenyl-uracils of the general formula (II) 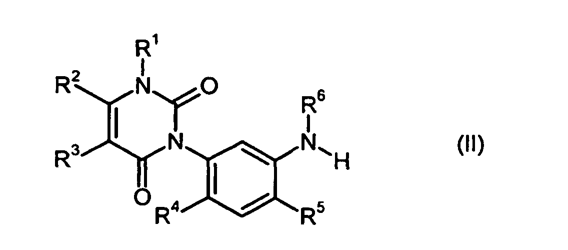 in which
R¹, R², R³, R⁴, R⁵ and R⁶ are each as defined in any of claims 1 to 4,
are reacted with acylating agents of the general formula (III) 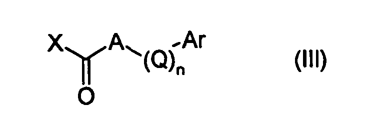 in which
n, A, Ar and Q are each as defined in any of claims 1 to 4, and
X represents halogen,
if appropriate in the presence of a reaction auxiliary and if appropriate in the presence of a diluent,
and, if appropriate, electrophilic or nucleophilic reactions or oxidations or reductions within the scope of the definition of the substituents are subsequently carried out in a customary manner.

6. The use of at least one substituted acylaminophenyl-uracil as claimed in any of claims 1 to 4 for controlling undesired plants.

7. A herbicidal which composition, which comprises at least one substituted acylaminophenyl-uracil as claimed in any of claims 1 to 4 and customary extenders.

## Revendications

1. Acylaminophényl-uraciles substitués de formule générale (I) 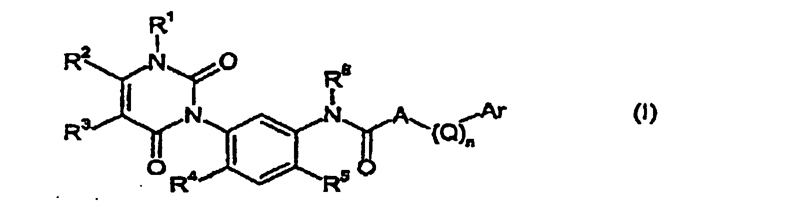 dans laquelle
n vaut 0 ou 1,
A représente un alcanediyle (alkylène) présentant 1 à 6 atomes de carbone éventuellement substitué à chaque fois par un substituant halogène, ou un cycloalcanediyle présentant 3 à 6 atomes de carbone ou, dans le cas où n vaut 1, représente aussi une liaison simple, ou est relié à Ar par l'intermédiaire d'un groupement alcanediyle présentant 1 à 3 atomes de carbone,
Ar représente un groupe phényle, naphtyle ou benzyle substitué à chaque fois, les substituants étant à prendre parmi les suivants :
nitro, cyano, fluor, chlore, brome, méthyle, éthyle, n- ou i-propyle, n-, i-, s- ou t- butyle, trifluorométhyle, méthoxy, éthoxy, n- ou i-propoxy, difluorométhoxy, trifluorométhoxy, méthylthio, éthylthio, n- ou i- propylthio, difluorométhylthio, trifluorométhylthio, méthylsulfinyle, éthylsulfinyle, n- ou i-propylsulfinyle, trifluorométhylsulfinyle, méthylsulfonyle, éthylsulfonyle, n- ou i- propylsulfonyle, trifluorométhylsulfonyle, méthoxycarbonyle, éthoxycarbonyle, n- ou i- propoxycarbonyle,
Q représente O (oxygène), S (soufre), SO, SO₂, NH ou N-alkyle en C₁-C₄,
R¹ représente un atome d'hydrogène, un groupe amino ou un groupe alkyle présentant 1 à 6 atomes de carbone substitué par un substituant cyano, carboxy, halogène, alkoxy en C₁-C₄ ou (alkoxy en C₁-C₄)carbonyle,
R² représente un groupe carboxy, cyano, carbamoyle, thiocarbamoyle ou un groupe alkyle ou alkoxycarbonyle présentant à chaque fois 1 à 6 atomes de carbone dans les groupes alkyle, substitué par un substituant cyano, halogène ou alkoxy en C₁-C₄,
R³ représente un atome d'hydrogène, un atome d'halogène ou un groupe alkyle présentant 1 à 6 atomes de carbone éventuellement substitué par un substituant halogène,
R⁴ représente un atome d'hydrogène, un groupe cyano, carbamoyle, thiocarbamoyle ou halogène,
R⁵ représente un groupe cyano, carbamoyle, thiocarbamoyle, halogène, ou un groupe alkyle ou alkoxy présentant à chaque fois 1 à 4 atomes de carbone éventuellement substitué à chaque fois par un substituant halogène, et
R⁶ représente un groupe alkylsulfonyle présentant 1 à 6 atomes de carbone éventuellement substitué à chaque fois par un substituant halogène,

2. Acylaminophényl-uraciles substitués selon la revendication 1, **caractérisés en ce que**
n vaut 0 ou 1,
A représente un groupe méthylène, éthane-1,1-diyle (éthylidène), éthane-1,2-diyle (diméthylène), propane-1,1-diyle (propylidène), propane-1,2-diyle ou propane-1,3-diyle (triméthylène), cyclopropane-1,1-diyle, cyclopropane-1,2-diyle, cyclobutane-1,1-diyle, cyclopentane-1,1-diyle ou cyclohexane-1,1-diyle éventuellement substitué à chaque fois par un substituant fluor et/ou chlore ou, dans le cas où n vaut 1, représente aussi une liaison simple, ou est lié à Ar par l'intermédiaire d'un groupement 1,2-éthanediyle-(diméthylène),
Ar représente un groupe phényle, naphtyle ou benzyle substitué à chaque fois, les substituants étant à prendre parmi les suivants :
nitro, cyano, fluor, chlore, brome, méthyle, éthyle, n- ou i-propyle, n-, i-, s- ou t- butyle, trifluorométhyle, méthoxy, éthoxy, n- ou i-propoxy, difluorométhoxy, trifluorométhoxy, méthylthio, éthylthio, n- ou i- propylthio, difluorométhylthio, trifluorométhylthio, méthylsulfinyle, éthylsulfinyle, n- ou i-propylsulfinyle, trifluorométhylsulfinyle, méthylsulfonyle, éthylsulfonyle, n- ou i- propylsulfonyle, trifluorométhylsulfonyle, méthoxycarbonyle, éthoxycarbonyle, n- ou i- propoxycarbonyle,
Q représente O (oxygène), S (soufre), SO, SO₂, NH ou N(méthyle),
R¹ représente un atome d'hydrogène, un groupe amino ou un groupe méthyle ou éthyle éventuellement substitué à chaque fois par un substituant cyano, fluor, chlore, méthoxy ou éthoxy,
R² représente un groupe carboxy, cyano, carbamoyle, thiocarbamoyle ou un groupe méthyle, éthyle, n- ou i-propyle, méthoxycarbonyle ou éthoxycarbonyle éventuellement substitué à chaque fois par un substituant cyano, fluor, chlore, méthoxy ou éthoxy,
R³ représente un atome d'hydrogène, de fluor, de chlore, de brome ou un groupe méthyle, éthyle, n- ou i-propyle éventuellement substitué à chaque fois par un substituant fluor et/ou chlore,
R⁴ représente un atome d'hydrogène, un groupe cyano, carbamoyle, thiocarbamoyle, fluor ou chlore,
R⁵ représente un groupe cyano, carbamoyle, thiocarbamoyle, fluor, chlore, brome, ou un groupe méthyle, éthyle, n- ou i-propyle, méthoxy, éthoxy, n- ou i-propoxy éventuellement substitué à chaque fois par un substituant fluor et/ou chlore,
R⁶ représente un groupe méthylsulfonyle, éthylsulfonyle, n- ou i-propylsulfonyle ou n-, i-, s- ou t-butylsulfonyle éventuellement substitué à chaque fois par un substituant fluor et/ou chlore.

3. Acylaminophényl-uraciles substitués selon la revendication 1, **caractérisés en ce que**
n vaut 1,
A représente un groupe méthylène, éthane-1,1-diyle (éthylidène) ou éthane-1,2-diyle (diméthylène),
Ar représente un groupe phényle, naphtyle ou benzyle substitué à chaque fois, les substituants étant à prendre parmi les suivants :
nitro, cyano, fluor, chlore, brome, méthyle, éthyle, n- ou i-propyle, n-, i-, s- ou t- butyle, trifluorométhyle, méthoxy, éthoxy, n- ou i-propoxy, difluorométhoxy, trifluorométhoxy, méthylthio, éthylthio, n- ou i- propylthio, difluorométhylthio, trifluorométhylthio, méthylsulfinyle, éthylsulfinyle, n- ou i-propylsulfinyle, trifluorométhylsulfinyle, méthylsulfonyle, éthylsulfonyle, n- ou i- propylsulfonyle, trifluorométhylsulfonyle, méthoxycarbonyle, éthoxycarbonyle, n- ou i- propoxycarbonyle,
Q représente O (oxygène),
R¹ représente un atome d'hydrogène, un groupe amino ou méthyle,
R² représente un groupe trifluorométhyle,
R³ représente un atome d'hydrogène, un atome de chlore ou un groupe méthyle,
R⁴ représente un atome d'hydrogène, de fluor ou de chlore,
R⁵ représente un groupe cyano, carbamoyle, thiocarbamoyle, chlore ou brome, et
R⁶ représente un groupe méthylsulfonyle, éthylsulfonyle, n- ou i-propylsulfonyle ou n-, i-, s- ou t-butylsulfonyle éventuellement substitué à chaque fois par un substituant fluor et/ou chlore.

4. Acylaminophényl-uraciles substitués selon la revendication 1, **caractérisés en ce que**
n vaut 0,
A représente un groupe méthylène, éthane-1,1-diyle (éthylidène) ou éthane-1,2-diyle (diméthylène),
Ar représente un groupe phényle ou naphtyle à chaque fois substitué, les substituants étant à prendre parmi les suivants :
nitro, cyano, fluor, chlore, brome, méthyle, éthyle, n- ou i-propyle, n-, i-, s- ou t- butyle, trifluorométhyle, méthoxy, éthoxy, n- ou i-propoxy, difluorométhoxy, trifluoroxméthoxy, méthylthio, éthylthio, n- ou i- propylthio, difluorométhylthio, trifluorométhylthio, méthylsulfinyle, éthylsulfinyle, n- ou i-propylsulfinyle, trifluorométhylsulfinyle, méthylsulfonyle, éthylsulfonyle, n- ou i- propylsulfonyle, trifluorométhylsulfonyle, méthoxycarbonyle, éthoxycarbonyle, n- ou i- propoxycarbonyle,
R¹ représente un atome d'hydrogène, un groupe amino ou méthyle,
R² représente un groupe trifluorométhyle,
R³ représente un atome d'hydrogène, un atome de chlore ou un groupe méthyle,
R⁴ représente un atome d'hydrogène, de fluor ou de chlore,
R⁵ représente un groupe cyano, carbamoyle, thiocarbamoyle, chlore ou brome, et
R⁶ représente un groupe méthylsulfonyle, éthylsulfonyle, n- ou i-propylsulfonyle ou n-, i-, s- ou t-butylsulfonyle éventuellement substitué à chaque fois par un substituant fluor et/ou chlore.

5. Procédé pour la préparation d'acylaminophényluraciles substitués selon l'une des revendications 1 à 4, **caractérisé en ce qu'**on transforme les aminophényl-uraciles de formule générale (II) 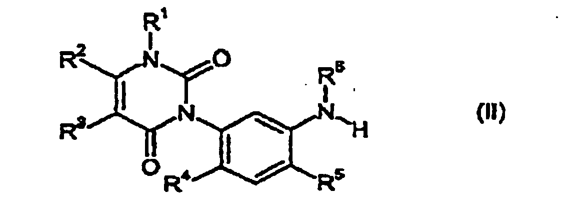 dans laquelle
R¹, R², R³, R⁴, R⁵ et R⁶ possèdent la signification donnée à l'une des revendications 1 à 4,
à l'aide d'agents d'acylation de formule générale (III) 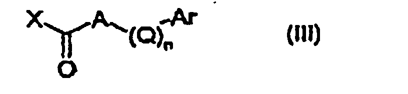 dans laquelle
n, A, Ar et Q possèdent la signification donnée à l'une des revendications 1 à 4
X représente un atome d'halogène,
éventuellement en présence d'un agent auxiliaire de réaction et éventuellement en présence d'un diluant,
et éventuellement on met en oeuvre ensuite, dans le cadre de la définition des substituants, des réactions d'oxydation ou de réduction électrophiles ou nucléophiles.

6. Utilisation d'au moins un acylaminophényl-uracile substitué selon l'une des revendications 1 à 4 pour la lutte contre des plantes indésirables.

7. Agents herbicides **caractérisés par** la teneur en au moins un acylaminophényl-uracile substitué selon l'une des revendications 1 à 4 et des diluants usuels.
